# EUROPEAN PATENT APPLICATION

(11) **EP 4 550 695 A1**
(43) Date of publication of application: **07.05.2025**
(21) Application number: 24208637.9
(22) Date of filing: 24.10.2024
(51) Int. Cl.: H04B 13/02

(54) **UNDERWATER OPTICAL COMMUNICATION SYSTEM AND UNDERWATER OPTICAL COMMUNICATION METHOD**

(30) Priority: 01.11.2023 JP 2023188000
(71) Applicant: SHIMADZU CORPORATION, Kyoto-shi, Kyoto 604-8511 (JP)
(72) Inventor: NISHIMURA, Naoki, Kyoto-shi, Kyoto, 604-8511 (JP)
(74) Representative: Müller-Boré & Partner Patentanwälte PartG mbB

(57) **Abstract**

An underwater optical communication system (1) according to this invention includes a first optical communication device (10) arranged underwater; a second optical communication device (20) being provided to a moving body (3) for moving underwater, and performing bidirectional optical communication between the second optical communication device and the first optical communication device (10); and a control device (30) for presenting at least one of a recommended relative position (41) and an unrecommended relative position (42) of at least the second optical communication device (20) relative to the first optical communication device (10) in the optical communication based on disturbance light.

## Description

### BACKGROUND OF THE INVENTION

### Field of the Invention

The present invention relates to an underwater optical communication system and an underwater optical communication method.

### Description of the Background Art

Underwater optical communication systems include a first optical communication device and a second optical communication device are known in the art. Such a system is disclosed in International Publication No. WO2021-090481, for example.

The above International Publication No. WO2021-090481 discloses an underwater optical communication system including a first optical communication device and a second optical communication device. The first optical communication device is provided to a fixed body installed on a water bottom. The second optical communication device is provided to a moving body that moves underwater. The underwater optical communication system is configured to be able to perform optical communication between the first optical communication device and the second optical communication device by receiving communication light emitted from the first optical communication device by using the second optical communication device and by receiving communication light emitted from the second optical communication device by using the first optical communication device.

However, in the underwater optical communication system as disclosed in the above International Publication No. WO2021-090481, a light receiver receives not only the communication light but also disturbance light such as sunlight. If the light receiver receives high-intensity disturbance light, the high-intensity disturbance light superimposed on the communication light may deteriorate communication quality or cause communication failure. For this reason, it is desired to reduce influence of disturbance light such as sunlight in underwater optical communication.

### SUMMARY OF THE INVENTION

The present invention is intended to solve the above problem, and one object of the present invention is to provide an underwater optical communication system and an underwater optical communication method capable of reducing influence of disturbance light such as sunlight during underwater optical communication.

In order to attain the aforementioned object, an underwater optical communication system according to a first aspect of the present invention includes a first optical communication device being arranged underwater, and including a first light emitter and a first light receiver; a second optical communication device being provided to a moving body for moving underwater, including a second light emitter and a second light receiver, and performing bidirectional optical communication between the second optical communication device and the first optical communication device; and a control device for presenting at least one of a recommended relative position and an unrecommended relative position of at least the second optical communication device relative to the first optical communication device in the optical communication based on disturbance light.

An underwater optical communication method according to a second aspect of the present invention is a method for performing optical communication between a first optical communication device and a second optical communication device underwater, the method including acquiring information on disturbance light; and presenting at least one of a recommended relative position and an unrecommended relative position of at least the second optical communication device relative to the first optical communication device based on the acquired information on the disturbance light.

In the underwater optical communication system according to the first aspect and the underwater optical communication method according to the second aspect of the present invention, at least one of a recommended relative position and an unrecommended relative position of at least the second optical communication device relative to the first optical communication device in the optical communication is presented based on disturbance light. Accordingly, since users can recognize at least one of the recommended relative position and the unrecommended relative position in the underwater optical communication, if the second optical communication device is not positioned at the recommended relative position, the moving body including the second optical communication device can be moved to the recommended relative position. Consequently, good optical communication can be performed between the second optical communication device and the first optical communication device. Also, in a case in which the moving body including the second optical communication device may enter the unrecommended relative position, users can avoid the second optical communication device from entering the unrecommended relative position by changing a moving direction of the moving body. Consequently, it is possible to prevent deterioration of communication quality and occurrence of interruption of communication between the second optical communication device and the first optical communication device in the water. Therefore, it is possible to reduce influence of disturbance light such as sunlight during underwater optical communication.

### BRIEF DESCRIPTION OF THE DRAWINGS

FIG. 1 is a block diagram showing an exemplary underwater optical communication system according to one embodiment.
FIG. 2 is a schematic view showing the exemplary underwater optical communication system according to the one embodiment.
FIG. 3 is a schematic view showing an exemplary first optical communication device according to the one embodiment.
FIG. 4 is a schematic view showing an exemplary virtual three-dimensional space according to the one embodiment.
FIG. 5 is a schematic view showing another exemplary virtual three-dimensional space according to the one embodiment.
FIG. 6 is a flowchart illustrating processing of presenting a recommended relative position and an unrecommended relative position of a second optical communication device relative to the first optical communication device according to the one embodiment.

### DESCRIPTION OF THE PREFERRED EMBODIMENT

Embodiments embodying the present invention will be described with reference to the drawings.

### (Underwater Optical Communication System)

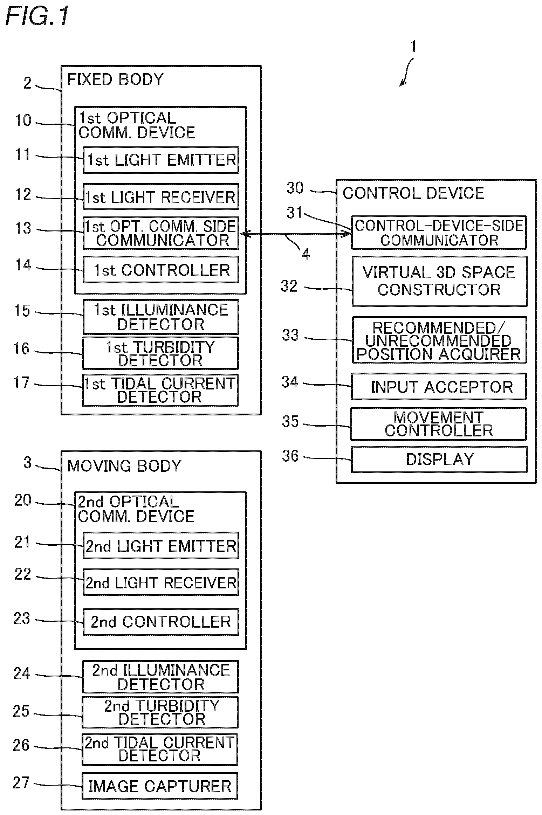

The following description describes an underwater optical communication system 1 according to one embodiment with reference to FIGS. 1 to 5. As shown in FIG. 1, the underwater optical communication system 1 includes a first optical communication device 10, a first illuminance detector 15, and a first turbidity detector 16, a first tidal current detector 17, a second optical communication device 20, a second illuminance detector 24, a second turbidity detector 25, a second tidal current detector 26, and a control device 30. Here, the first illuminance detector 15 and the second illuminance detector 24 are examples of an "illuminance detector" in the claims. Also, the first turbidity detector 16 and the second turbidity detector 25 are examples of a "turbidity detector" in the claims. Also, the first tidal current detector 17 and the second tidal current detector 26 are examples of a "tidal current detector" in the claims.

Here, underwater or in the water refers to in seawater, in lakewater, and the like in this specification, for example. Also, the "water" in the water refers to seawater, freshwater, brackish water, and the like, for example. Also, a water bottom refers to a sea bottom, a lake bottom, and the like, for example. Also, on or above the water refers to on or above the seawater, on or above the lakewater and the like, for example.

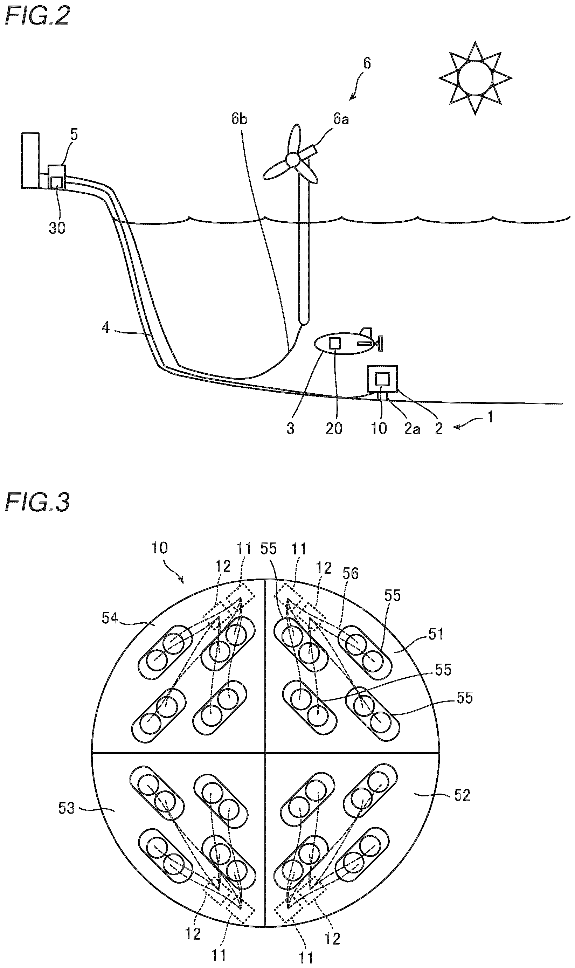

As shown in FIG. 2, the first optical communication device 10 and the second optical communication device 20 are optical communication devices arranged in the water. The underwater optical communication system 1 is configured to be able to perform optical communication between the first optical communication device 10 and the second optical communication device 20 by receiving communication light emitted from the first optical communication device 10 by using the second optical communication device 20. Also, the underwater optical communication system is configured to be able to perform optical communication between the second optical communication device 20 and the first optical communication device 10 by receiving communication light emitted from the second optical communication device 20 by using the first optical communication device 10.

The control device 30 is provided to a base station 5 on land. The first communication device communicates with the control device 30 on land through an underwater cable 4. The control device 30 and the first optical communication device 10 are configured to be able to communicate with each other through the underwater cable 4. As described later, the control device 30 constructs a virtual three-dimensional space 40 on which a real-time real environment in the water is reflected, and presents the recommended relative position 41 and the unrecommended relative position 42 of the second optical communication device 20 relative to the first optical communication device 10 in the optical communication based on disturbance light in the virtual three-dimensional space 40.

### (First Optical Communication Device, First Illuminance Detector, First Turbidity Detector, and First Tidal Current Detector)

As shown in FIG. 1, the first optical communication device 10, the first illuminance detector 15, the first turbidity detector 16 and the first tidal current detector 17 are arranged in the water. Specifically, the first optical communication device 10, the first illuminance detector 15, the first turbidity detector 16, and the first tidal current detector 17 are included in a fixed body 2 fixedly arranged in the water. The fixed body 2 is installed on the water bottom through a retainer 2a to be fixedly arranged in the water. Also, the first optical communication device 10, the first illuminance detector 15, the first turbidity detector 16 and the first tidal current detector 17 are configured to be able to communicate with each other wirelessly or through wires.

The first optical communication device 10 includes first light emitters 11, first light receivers 12, a first optical communication side communicator 13, and a first controller 14. As shown in FIG. 3, the first optical communication device 10 illustratively has a hemispherical shape whose area is divided into a first area 51, a second area 52, a third area 53 and a fourth area 54. The first area 51, the second area 52, the third area 53 and the fourth area 54 are orientated in directions different from each other. Each of the first area 51, the second area 52, the third area 53 and the fourth area 54 in the first optical communication device 10 includes one first light emitter 11 and one first light receiver 12. Each of the first area 51, the second area 52, the third area 53 and the fourth area 54 has four windows 55 orientated to face in directions different from each other, and output ends of optical fibers 56 extending from the first light emitter 11 and input ends of optical fibers 56 extending from the first light receiver 12 are arranged in each of the four windows 55.

The first optical communication device 10 is configured to be able to transmit a plurality of types of communication light in a plurality of directions by using the first light emitters 11, and to be able to receive the plurality of types of communication light in a plurality of directions by using the first light receivers 12. Accordingly, the first controller 14 can acquire relative position information on the second optical communication device 20 relative to the first optical communication device 10 based on light receiving elements of the first light receiver 12 that receives the communication light output from a second light emitter 21 of the second optical communication device 20.

As shown in FIG. 1, the first light emitters 11 are configured to emit laser light as the communication light. The first light emitters 11 include LD (Laser Diode) emitters including semiconductor light emitting elements, for example. Laser light is green or blue visible light, which is unlikely to diminish in the water, for example.

The first light receivers 12 are configured to receive the communication light in the water. Each of the first light detectors 12 includes a plurality of light receiving elements and a photomultiplier tube.

The first optical communication side communicator 13 is configured to be able to communicate with the control device 30 through the underwater cable 4.

The first controller 14 is configured to control light emission by the first light emitters 11 and light reception by the first light receivers 12. Also, the first controller 14 is configured to transmit electrical signals converted from the communication light received by the first light receivers 12 to the control device 30 through the underwater cable 4. Also, the first controller 14 is configured to acquire the relative position information on the second optical communication device 20 relative to the first optical communication device 10 based on the light receiving elements of the first light receiver 12 that receives the communication light output from the second light emitter 21 of the second optical communication device 20, and to be able to transmit the acquired relative position information on the second optical communication device 20 relative to the first optical communication device 10 to the control device 30 through the underwater cable 4. The first controller 14 is a control circuit including a processor such as a CPU (Central Processing Unit), and a memory.

The first illuminance detector 15 is configured to detect illuminances of the disturbance light such as sunlight, illumination light from other moving bodies other than a moving body 3 and structures, and the like. Also, the first illuminance detector 15 is configured to detect the illuminances of the disturbance light in a plurality of directions different from each other. The first illuminance detector 15 is arranged in proximity to the first optical communication device 10 in the fixed body 2, and is configured to detect the intensities of illuminances (light intensities) of the disturbance light in proximity to the first optical communication device 10 in a plurality of directions different from each other. The illuminances of the disturbance light detected by the first illuminance detector 15 are transmitted to the first optical communication device 10, and are transmitted from the first optical communication device 10 to the control device 30 through the underwater cable 4. The first illuminance detector 15 includes a plurality of illuminometers. The plurality of illuminometers are orientated to detect the illuminances of the disturbance light in a plurality of directions different from each other. Here, the first illuminance detector 15 may be a single illuminometer configured to be able to change its illuminance detection direction.

The first turbidity detector 16 is configured to detect turbidity in the water. The first turbidity detector 16 is arranged in proximity to the first optical communication device 10 in the fixed body 2, and is configured to detect turbidity in the water (degree of water turbidity) in proximity to the first optical communication device 10. The turbidity in the water detected by the first turbidity detector 16 is transmitted to the first optical communication device 10, and is transmitted from the first optical communication device 10 to the control device 30 through the underwater cable 4. The first turbidity detector 16 includes one or more turbidimeters, for example.

The first tidal current detector 17 is configured to detect a tidal current in the water. The first tidal current detector 17 is arranged in proximity to the first optical communication device 10 in the fixed body 2, and is configured to detect the tidal current in the water (seawater current) in proximity to the first optical communication device 10. The tidal current in the water detected by the first tidal current detector 17 is transmitted to the first optical communication device 10, and is transmitted from the first optical communication device 10 to the control device 30 through the underwater cable 4. The first tidal current detector 17 includes one or more tidal current meters, for example.

### (Second Optical Communication Device, Second Illuminance Detector, Second Turbidity Detector, and Second Tidal Current Detector)

The second optical communication device 20, the second illuminance detector 24, the second turbidity detector 25 and the second tidal current detector 26 are arranged in the water. Specifically, the second optical communication device 20, the second illuminance detector 24, the second turbidity detector 25, and the second tidal current detector 26 are included in the moving body 3 that moves in the water as shown in FIG. 2. The moving body 3 includes an AUV (Autonomous Underwater Vehicle), for example. The moving body 3 includes a propeller, such as a screw propeller, and can move in the water. The moving body 3 includes an image capturer 27 (see FIG. 1) for capturing images of a to-be-observed subject 6 in the water. The image capturer 27 is a camera, for example. For example, the to-be-observed subject 6 is a component, such as a power cable 6b in an offshore wind power generation facility 6a, or an aquaculture facility. Also, the second optical communication device 20, the second illuminance detector 24, the second turbidity detector 25 and the second tidal current detector 26 are configured to be able to communicate with each other wirelessly or through wires.

As shown in FIG. 1, the second optical communication device 20 includes the second light emitters 21, a second light receiver 22, and a second controller.

The second light emitter 21 is configured to emit laser light as the communication light. The second light emitter 21 includes an LD emitter including a semiconductor light emitting elements, for example. Laser light is green or blue visible light, which is unlikely to attenuate in the water, for example.

The second light receiver 22 is configured to receive the communication light in the water. The second light receiver 22 includes a plurality of light receiving elements and a photomultiplier tube.

The second controller 23 is configured to control light emission by the second light emitter 21 and light reception by the second light receiver 22. Also, the second controller 23 is configured to convert image data acquired by the image capturer 27 into optical signals, and to transmit the optical signals to the first optical communication device 10 through the communication light. The second controller 23 is a control circuit including a processor such as a CPU (Central Processing Unit), and a memory.

The second illuminance detector 24 is configured to detect illuminances of the disturbance light such as sunlight, illumination light from other moving bodies other than the moving body 3 and structures, and the like. Also, the second illuminance detector 24 is configured to detect the illuminances of the disturbance light in a plurality of directions different from each other. The second illuminance detector 24 is arranged in the moving body 3, and is configured to detect the intensities of illuminances (light intensities) of the disturbance light in proximity to the second optical communication device 20 in a plurality of directions different from each other. The illuminances of the disturbance light detected by the second illuminance detector 24 are transmitted to the second optical communication device 20, and are converted into optical signals and transmitted from the second optical communication device 20 to the first optical communication device 10. The second illuminance detector 24 includes a plurality of illuminometers. The plurality of illuminometers are orientated to detect the illuminances of the disturbance light in a plurality of directions different from each other. Here, the second illuminance detector 24 may be a single illuminometer configured to be able to change its illuminance detection direction.

The second turbidity detector 25 is configured to detect turbidity in the water. The second turbidity detector 25 is arranged in the moving body 3, and is configured to detect turbidity in the water in proximity to the second optical communication device 20. The turbidity in the water detected by the second turbidity detector 25 is transmitted to the second optical communication device 20, and is converted into an optical signal and transmitted from the second optical communication device 20 to the first optical communication device 10. The second turbidity detector 25 includes one or more turbidimeters, for example.

The second tidal current detector 26 is configured to detect a tidal current in the water. The second tidal current detector 26 is arranged in the moving body 3, and is configured to detect a tidal current in the water in proximity to the second optical communication device 20. The tidal current in the water detected by the second tidal current detector 26 is transmitted to the second optical communication device 20, and is converted into an optical signal and transmitted from the second optical communication device 20 to the first optical communication device 10. The second tidal current detector 26 includes one or more tidal current meters, for example.

### (Control Device)

The control device 30 includes, for example, a PC (personal computer). The control device 30 includes a control-device-side communicator 31, a virtual three-dimensional space constructor 32, a recommended/unrecommended position acquirer 33, an input acceptor 34, and a display 35 and a movement controller 36.

The control-device-side communicator 31 is configured to be able to communicate with the first optical communication side communicator 13 through the underwater cable 4.

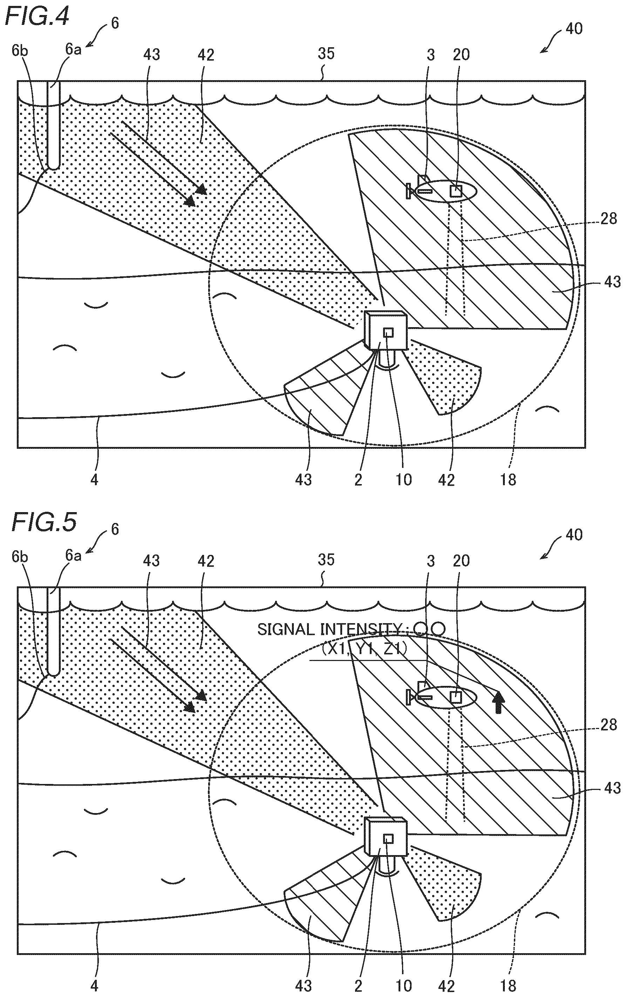

The virtual three-dimensional space constructor 32 is configured to construct the virtual three-dimensional space 40 (see FIG. 4) on which a real-time real environment is reflected. The virtual three-dimensional space 40 is a so-called digital twin. That is, in the virtual three-dimensional space 40, an underwater real space is reproduced including, in addition to the first optical communication device 10, the fixed body 2, the second optical communication device 20, the moving body 3, the to-be-observed subject 6 to be observed by the second optical communication device 20, and the like, an underwater environment such as a disturbance-light-influence direction 43 in the water, the degree of turbidity in the water and the tidal current. For example, the virtual three-dimensional space constructor 32 is constructed of a processor such as a GPU (Graphics Processing Unit) or a CPU (Central Processing Unit). The construction of the virtual three-dimensional space 40 by the virtual three-dimensional space constructor 32 will be described later.

The recommended/unrecommended position acquirer 33 is configured to acquire the recommended relative position 41 (see FIG. 4) and the unrecommended relative position 42 (see FIG. 4) of the second optical communication device 20 relative to the first optical communication device 10 in the virtual three-dimensional space 40 (see FIG. 4) in the optical communication between the first optical communication device 10 and the second optical communication device 20. Also, the recommended/unrecommended position acquirer 33 is configured to present the recommended relative position 41 and the unrecommended relative position 42 acquired of the second optical communication device 20 relative to the first optical communication device 10. That is, the recommended/unrecommended position acquirer 33 is configured to indicate the recommended relative position 41 and the unrecommended relative position 42 of the second optical communication device 20 relative to the first optical communication device 10 superimposed on the virtual three-dimensional space 40 in which the first optical communication device 10 and the second optical communication device 20 are displayed on the display 35. For example, the recommended/unrecommended position acquirer 33 is constructed of a processor such as a CPU (Central Processing Unit). The acquisition of the recommended relative position 41 and the unrecommended relative position 42 of the second optical communication device 20 relative to the first optical communication device 10 by the recommended/unrecommended position acquirer 33 will be described later.

The input acceptor 34 is configured to accept operating inputs from users. For example, the input acceptor 34 includes an input device such as a computer mouse, a keyboard, and the like.

The display 35 is configured to display the virtual three-dimensional space 40 constructed by the virtual three-dimensional space constructor 32. The display 35 includes a display device such as an LCD monitor, for example.

The movement controller 36 is configured to move the moving body 3 including the second optical communication device 20 through the underwater cable 4 and the first optical communication device 10. If the input acceptor 34 accepts an input of the position to which the second optical communication device 20 is to be moved in the virtual three-dimensional space 40 based on the presented at least one of the recommended relative position 41 and the unrecommended relative position 42 of at least the second optical communication device 20 relative to the first optical communication device 10, the movement controller 36 is configured to move the second optical communication device 20 based on the input position through the underwater cable 4 and the first optical communication device 10.

### (Construction of Virtual Three-Dimensional Space)

The following description the construction of the virtual three-dimensional space 40. As shown in FIG. 4, the virtual three-dimensional space constructor 32 constructs the virtual three-dimensional space 40, and displays the virtual three-dimensional space 40 on the display 35.

The virtual three-dimensional space constructor 32 arranges the first optical communication device 10, the fixed body 2, the second optical communication device 20, the moving body 3, and the to-be-observed subject 6 to be observed by the second optical communication device 20 in response to an operation of a user accepted by the input acceptor 34 in the virtual three-dimensional space 40 in which the underwater real space is reproduced. In addition, the virtual three-dimensional space constructor 32 arranges the first illuminance detector 15 (see FIG. 1), the first turbidity detector 16 (see FIG. 1), the first tidal current detector 17 (see FIG. 1), the second illuminance detector 24 (see FIG. 1), the second turbidity detector 25 (see FIG. 1), and the second tidal current detector 26 (see FIG. 1) in response to an operation of the user accepted by the input acceptor 34 in the virtual three-dimensional space 40 in which the underwater real space is reproduced. In this construction, the user specifies shapes, the numbers, positions, and the like of the aforementioned objects to be arranged by inputting them through the input acceptor 34. In addition, the user inputs absolute position information on the first optical communication device 10 included in the fixed body 2 in the virtual three-dimensional space 40 through the input acceptor 34. That is, the virtual three-dimensional space constructor 32 acquires the absolute position information on the first optical communication device 10.

The virtual three-dimensional space constructor 32 acquires the relative position information on the second optical communication device 20 relative to the first optical communication device 10 from the first optical communication device 10 through the underwater cable 4. The virtual three-dimensional space constructor 32 updates and indicates positions of the second optical communication device 20 and the moving body 3 in the virtual three-dimensional space 40 based on the absolute position information on the first optical communication device 10 and the relative position information on the second optical communication device 20 relative to the first optical communication device 10 to reflect current positions of the second optical communication device 20 and the moving body 3 on the virtual three-dimensional space 40.

The virtual three-dimensional space constructor 32 acquires the intensities of illuminances (light intensities) of the disturbance light in a plurality of different directions from each other detected by the first illuminance detector 15, and the intensities of illuminances (light intensities) of the disturbance light in a plurality of different directions from each other detected by the second illuminance detector 24 from the first optical communication device 10 through the underwater cable 4. The virtual three-dimensional space constructor 32 acquires an incident direction of the disturbance light that has a light intensity not smaller than a threshold as the disturbance-light-influence direction 43. Subsequently, the virtual three-dimensional space constructor 32 indicates the acquired disturbance-light-influence direction 43 in the virtual three-dimensional space 40.

Subsequently, the virtual three-dimensional space constructor 32 updates the disturbance-light-influence direction 43 and indicates the updated disturbance-light-influence direction in the virtual three-dimensional space 40 based on the newly acquired intensities of illuminances (light intensities) of the disturbance light in a plurality of different directions from each other detected by the first illuminance detector 15, and the newly acquired intensities of illuminances (light intensities) of the disturbance light in a plurality of different directions from each other detected by the second illuminance detector 24.

The virtual three-dimensional space constructor 32 acquires turbidity in the water in proximity to the first optical communication device 10 detected by the first turbidity detector 16, and turbidity in the water in proximity to the second optical communication device 20 detected by the second turbidity detector 25 from the first optical communication device 10 through the underwater cable 4. The virtual three-dimensional space constructor 32 acquires an attenuation rate of the communication light based on the turbidity in the water, and acquires a signal intensity 44 of the communication light at coordinates in the virtual three-dimensional space 40 based on the attenuation rate of the communication light. Subsequently, the virtual three-dimensional space constructor 32 indicates the signal intensity 44 of the acquired signal intensity of the communication light in response to an operation of the user accepted by the input acceptor 34 in the virtual three-dimensional space 40 as shown in FIG. 5. As an exemplary indication, if the user makes a selection operation (click, or the like) through the input acceptor 34, the signal intensity 44 of the communication light at a position where a cursor 45 is position is indicated in the virtual three-dimensional space 40.

Subsequently, the virtual three-dimensional space constructor 32 updates the signal intensity 44 of the communication light based on the newly acquired turbidity in the water in proximity to the second optical communication device 20 detected by the first turbidity detector 16, and the newly acquired turbidity in the water in proximity to the second optical communication device 20 detected by the second turbidity detector 25, and indicates the updated signal intensity in the virtual three-dimensional space 40.

As shown in FIG. 4, the virtual three-dimensional space constructor 32 acquires a tidal current in the water in proximity to the first optical communication device 10 detected by the first tidal current detector 17, and a tidal current in the water in proximity to the second optical communication device 20 detected by the second tidal current detector 26 from the first optical communication device 10 through the underwater cable 4. Subsequently, the virtual three-dimensional space constructor 32 acquires a position of the to-be-observed subject 6, which will be moved by influences of the tidal currents on the power cable 6b in the offshore wind power generation facility 6a, based on the acquired tidal currents in the water, for example. Subsequently, the virtual three-dimensional space constructor 32 indicates the acquired position of the to-be-observed subject 6 in the virtual three-dimensional space 40.

Subsequently, the virtual three-dimensional space constructor 32 updates the position of the to-be-observed subject 6 based on the newly acquired tidal current in the water in proximity to the first optical communication device 10 detected by the first tidal current detector 17, and the newly acquired tidal current in the water in proximity to the second optical communication device 20 detected by the second tidal current detector 26, and indicates the updated position of the to-be-observed subject in the virtual three-dimensional space 40.

In addition, the virtual three-dimensional space constructor 32 indicates a light area of the communication light 18 emitted by the first light emitters 11 of the first optical communication device 10, and a light area 28 of the communication light emitted by the second light emitter 21 of the second optical communication device 20 in the virtual three-dimensional space 40.

### (Acquisition of Recommended and Unrecommended Relative Positions of Second Optical Communication Device Relative to First Optical Communication Device)

The following description describes acquisition of the recommended relative position 41 and the unrecommended relative position 42 of the second optical communication device 20 relative to the first optical communication device 10. The recommended/unrecommended position acquirer 33 acquires the recommended relative position 41 and the unrecommended relative position 42 of the second optical communication device 20 relative to the first optical communication device 10, and superimposes the recommended relative position 41 and the unrecommended relative position 42 of the second optical communication device 20 relative to the first optical communication device 10 on the virtual three-dimensional space 40 displayed on the display 35.

Here, the recommended relative position 41 of the second optical communication device 20 relative to the first optical communication device 10 refers to a relative position of the second optical communication device 20 relative to the first optical communication device 10 at which influences of disturbance light such as sunlight, turbidity in the water and tidal currents are suppressed so that optical communication between the first optical communication device 10 and the second optical communication device 20 can be stably and continuously performed.

The recommended/unrecommended position acquirer 33 acquires the recommended relative position 41 of the second optical communication device 20 relative to the first optical communication device 10 based on the disturbance-light-influence direction 43 in the virtual three-dimensional space 40, the signal intensity 44 of the communication light at the coordinates of the virtual three-dimensional space 40, and a tidal current in the virtual three-dimensional space 40. Specifically, the recommended/unrecommended position acquirer 33 acquires, as the recommended relative position 41 of the second optical communication device 20 relative to the first optical communication device 10, a position that satisfies conditions that an angle that is formed by the disturbance-light-influence direction 43 and a direction of the second optical communication device 20 relative to the first optical communication device 10 falls within a set range, that the signal intensity 44 of the communication light is not smaller than a set value, and that an angle that is formed by a direction of the tidal current and a moving direction of the moving body 3 falls within a set range. The recommended/unrecommended position acquirer 33 superimposes the recommended relative position 41 of the second optical communication device 20 relative to the first optical communication device 10 on the virtual three-dimensional space 40 displayed on the display 35.

Also, the unrecommended relative position 42 of the second optical communication device 20 relative to the first optical communication device 10 refers to a relative position of the second optical communication device 20 relative to the first optical communication device 10 at which influences of disturbance light such as sunlight, turbidity in the water and tidal currents are large so that it is difficult to stably and continuously perform optical communication between the first optical communication device 10 and the second optical communication device 20. An example of the unrecommended relative position 42 of the second optical communication device 20 relative to the first optical communication device 10 is a position of the second optical communication device 20 relative to the first optical communication device 10 at which a position of the first optical communication device 10, a position of the second optical communication device 20, and a position of the sun are aligned on the same line.

The recommended/unrecommended position acquirer 33 acquires the unrecommended relative position 42 of the second optical communication device 20 relative to the first optical communication device 10 based on the disturbance-light-influence direction 43 in the virtual three-dimensional space 40, the signal intensity 44 of the communication light at the coordinates of the virtual three-dimensional space 40, and a tidal current in the virtual three-dimensional space 40. Specifically, the recommended/unrecommended position acquirer 33 acquires, as the unrecommended relative position 42 of the second optical communication device 20 relative to the first optical communication device 10, a position that satisfies conditions that the angle that is formed by the disturbance-light-influence direction 43 and the direction of the second optical communication device 20 relative to the first optical communication device 10 falls out of the set range, that the signal intensity 44 of the communication light is smaller than the set value, and that the angle that is formed by the direction of the tidal current and the moving direction of the moving body 3 falls out of the set range. The recommended/unrecommended position acquirer 33 superimposes the unrecommended relative position 42 of the second optical communication device 20 relative to the first optical communication device 10 on the virtual three-dimensional space 40 displayed on the display 35.

### (Movement of Moving Body Including Second Optical Communication Device)

In a case in which the user will move the moving body 3 including the second optical communication device 20 to the recommended relative position 41 of the second optical communication device 20 relative to the first optical communication device 10 presented in the virtual three-dimensional space 40, the user specifies the position to which the user will move the moving body 3 in the virtual three-dimensional space 40 through the input acceptor 34. Also, in a case in which the moving body 3 including the second optical communication device 20 may enter the unrecommended relative position 42 of the second optical communication device 20 relative to the first optical communication device 10 presented in the virtual three-dimensional space 40, the user specifies the position to which the user will move the moving body 3 in the virtual three-dimensional space 40 through the input acceptor 34. The movement controller 36 controls movement of the moving body 3 including the second optical communication device 20 based on the specified position through the underwater cable 4 and the first optical communication device 10.

### (In Interruption of Bidirectional Optical Communication)

The movement controller 36 controls, if the bidirectional optical communication between the first optical communication device 10 and the second optical communication device 20 is interrupted, movement of the second optical communication device 20 to a position that is set to allow the bidirectional optical communication between the first optical communication device 10 and the second optical communication device. If the bidirectional optical communication between the first optical communication device 10 and the second optical communication device 20 is interrupted by movement of moving body 3 including the second optical communication device 20 to the unrecommended relative position 42 or a position corresponding to the unrecommended relative position 42 under construction of the virtual three-dimensional space 40 or after construction of the virtual three-dimensional space 40 by the virtual three-dimensional space constructor 32, the movement controller controls movement of the second optical communication device 20 to the position that is set to allow the bidirectional optical communication between the first optical communication device 10 and the second optical communication device. That is, if the bidirectional optical communication between the first optical communication device 10 and the second optical communication device 20 is interrupted, the movement controller 36 moves the moving body 3 including the second optical communication device 20 to the position that is set to allow the bidirectional optical communication between the first optical communication device 10 and the second optical communication device.

Here, although it is difficult to stably and continuously perform optical communication between the first optical communication device 10 and the second optical communication device 20 at the unrecommended relative position 42 or the position corresponding to the unrecommended relative position 42, optical communication between the first optical communication device 10 and the second optical communication device 20 can be performed in some cases if an amount of data is relatively small. For this reason, if the movement controller 36 cannot acquire the relative position information on the second optical communication device 20 relative to the first optical communication device 10 through the first optical communication device 10, the movement controller moves the moving body 3 including the second optical communication device 20 to the position that is set to allow the bidirectional optical communication between the first optical communication device 10 and the second optical communication device by transmitting a minimum amount of data from the first optical communication device 10 to the second optical communication device 20 through optical communication. The position that is set to allow the bidirectional optical communication between the first optical communication device 10 and the second optical communication device may be a previously set position, or the recommended relative position 41 of the second optical communication device 20 relative to the first optical communication device 10 presented in the virtual three-dimensional space 40.

Here, if the movement controller 36 cannot acquire the relative position information on the second optical communication device 20 relative to the first optical communication device 10 through the first optical communication device 10, the movement controller may be configured to move the moving body 3 including the second optical communication device 20 to the position that is set to allow the bidirectional optical communication between the first optical communication device 10 and the second optical communication device by performing acoustic communication between the first optical communication device 10 and the second optical communication device 20. Alternatively, if the bidirectional optical communication between the first optical communication device 10 and the second optical communication device 20 is interrupted, the moving body 3 including the second optical communication device 20 may autonomously move to the position that is set to allow the bidirectional optical communication between the first optical communication device 10 and the second optical communication device instead of control executed by the movement controller 36.

### (Processing of Presenting Recommended and Unrecommended Relative Positions)

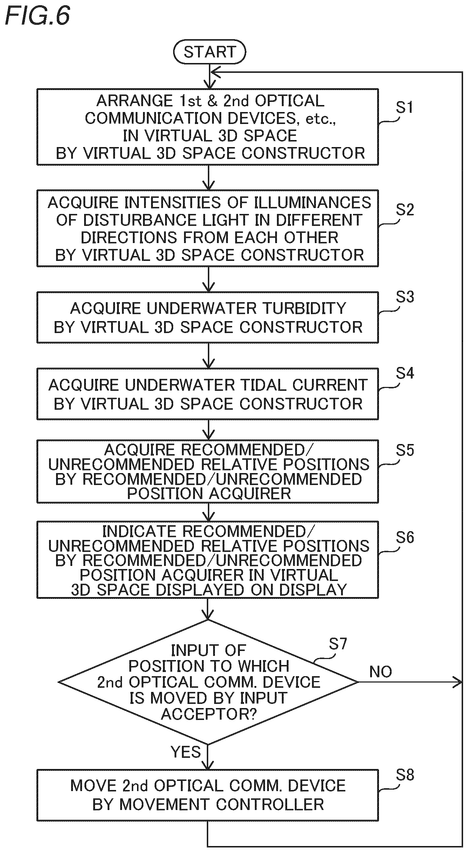

The following description describes processing of presenting the recommended relative position 41 and the unrecommended relative position 42 of the second optical communication device 20 relative to the first optical communication device 10 executed by the control device 30 according to the one embodiment with reference to FIG. 6. Here, order of processing steps can be changed, or the processing steps simultaneously executed as long as they do not conflict with each other.

In step S1, the virtual three-dimensional space constructor 32 arranges the first optical communication device 10, the fixed body 2, the second optical communication device 20, the moving body 3, the to-be-observed subject 6 to be observed by the second optical communication device 20, the first illuminance detector 15, the first turbidity detector 16, the first tidal current detector 17, the second illuminance detector 24, the second turbidity detector 25, and the second tidal current detector 26 in response to an operation of a user accepted by the input acceptor 34 in the virtual three-dimensional space 40 in which the underwater real space is reproduced. Subsequently, the procedure goes to step S2.

In step S2, the virtual three-dimensional space constructor 32 acquires intensities of illuminances (light intensities) of the disturbance light in a plurality of different directions from each other detected by the first illuminance detector 15 from the first optical communication device 10, and acquires the disturbance-light-influence direction 43 based on the acquired intensities of illuminances (light intensities) of the disturbance light in a plurality of different directions detected from each other. Also, the virtual three-dimensional space constructor 32 acquires intensities of the disturbance light such as sunlight (light intensities) by the second illuminance detector 24 from the second optical communication device 20 through the first optical communication device 10, and acquires the disturbance-light-influence direction 43 based on the acquired intensities of illuminances (light intensities) of the disturbance light in a plurality of different directions detected from each other. Subsequently, the procedure goes to step S3.

In step S3, the virtual three-dimensional space constructor 32 acquires turbidity in the water detected by the first turbidity detector 16 from the first optical communication device 10. Also, the virtual three-dimensional space constructor 32 acquires turbidity detected by the second turbidity detector 25 from the second optical communication device 20 through the first optical communication device 10. The virtual three-dimensional space constructor 32 acquires an attenuation rate of the communication light based on the turbidity in the water, and acquires a signal intensity 44 of the communication light at coordinates in the virtual three-dimensional space 40 based on the attenuation rate of the communication light. The process then proceeds to step S4.

In step S4, the virtual three-dimensional space constructor 32 acquires a tidal current in the water detected by the first tidal current detector 17 from the first optical communication device 10. Also, the virtual three-dimensional space constructor 32 acquires a tidal current detected by the second tidal current detector 26 from the second optical communication device 20 through the first optical communication device 10. Subsequently, the virtual three-dimensional space constructor 32 acquires a moved position of the to-be-observed subject 6, which will be moved by influences of the tidal currents, based on the acquired tidal currents in the water. Subsequently, the procedure goes to step S5.

In step S5, the recommended/unrecommended position acquirer 33 acquires the recommended relative position 41 and the unrecommended relative position 42 of the second optical communication device 20 relative to the first optical communication device 10 based on the disturbance-light-influence direction 43 in the virtual three-dimensional space 40, the signal intensity 44 of the communication light at the coordinates of the virtual three-dimensional space 40, and a tidal current in the virtual three-dimensional space 40. Subsequently, the procedure goes to step S6.

In step S6, the recommended/unrecommended position acquirer 33 superimposes the recommended relative position 41 and the unrecommended relative position 42 of the second optical communication device 20 relative to the first optical communication device 10 on the virtual three-dimensional space 40 displayed on the display 35. Subsequently, the procedure goes to step S7.

If the input acceptor 34 accepts an input of a position to which the second optical communication device 20 is to be moved in the virtual three-dimensional space 40 in step S7 (Yes in step S7), the procedure goes to step S8, and if the input acceptor 34 accepts no input of a position to which the second optical communication device 20 is to be moved in the virtual three-dimensional space 40 within a predetermined time period (No in step S7), the procedure goes to step S2.

In step S8, the movement controller 36 moves the second optical communication device 20 based on the input position accepted by using the first optical communication device 10 through the underwater cable 4. Subsequently, the procedure goes to step S2.

Operations of step S2 are step S8 is repeated for each control cycle.

### (Advantages of the Embodiment)

In this embodiment, the following advantages are obtained.

As described above, the underwater optical communication system 1 according to this embodiment includes a first optical communication device 10 being arranged underwater, and including first light emitters 11 and first light receivers 12; a second optical communication device 20 being provided to a moving body 3 for moving underwater, including a second light emitter 21 and a second light receiver 22, and performing bidirectional optical communication between the second optical communication device and the first optical communication device 10; and a control device 30 for presenting at least one of a recommended relative position 41 and an unrecommended relative position 42 of at least the second optical communication device 20 relative to the first optical communication device 10 in the optical communication based on disturbance light.

Accordingly, since users can recognize the recommended relative position 41 and the unrecommended relative position 42 in the underwater optical communication, if the second optical communication device 20 is not positioned at the recommended relative position 41, the moving body 3 including the second optical communication device 20 can be moved to the recommended relative position 41. Consequently, good optical communication can be performed between the second optical communication device 20 and the first optical communication device 10. Also, in a case in which the moving body 3 including the second optical communication device 20 may enter the unrecommended relative position 42, users can avoid the second optical communication device 20 from entering the unrecommended relative position 42 by changing a moving direction of the moving body 3. Consequently, it is possible to prevent deterioration of communication quality and occurrence of interruption of communication between the second optical communication device 20 and the first optical communication device 10 in the water. Therefore, it is possible to reduce influence of disturbance light such as sunlight during underwater optical communication.

Also, as described above, the underwater optical communication method according to this embodiment is a method for performing optical communication between a first optical communication device 10 and a second optical communication device 20 underwater, the method including acquiring information on disturbance light; and presenting at least one of a recommended relative position 41 and an unrecommended relative position 42 of at least the second optical communication device 20 relative to the first optical communication device 10 based on the acquired information on the disturbance light.

Accordingly, it is possible to provide an underwater optical communication method capable of reducing influence of disturbance light such as sunlight during underwater optical communication similar to the aforementioned underwater optical communication system 1.

In addition, following additional advantages can be obtained by the aforementioned embodiment added with configurations discussed below.

That is, in this embodiment, as described above, the control device 30 presents the recommended relative position 41 and the unrecommended relative position 42 of the second optical communication device 20 relative to the first optical communication device 10 in the optical communication based on light intensities of the disturbance light in a plurality of directions different from each other. Accordingly, since the disturbance-light-influence direction 43 of the disturbance light, which can cause deterioration of communication quality and occurrence of interruption of communication, can be acquired by detecting intensities of illuminances (light intensities) of the disturbance light in a plurality of specified directions, it is possible to accurately acquire the recommended relative position 41 and the unrecommended relative position 42 of the second optical communication device 20 relative to the first optical communication device 10. Consequently, it is possible to further reduce influence of disturbance light such as sunlight during optical communication in the water during underwater optical communication between the first optical communication device 10 and the second optical communication device 20.

In this embodiment, as described above, the control device is configured to move the second optical communication device 20 to a position that is operated based on the presented at least one of the recommended relative position 41 and the unrecommended relative position 42 of at least the second optical communication device 20 relative to the first optical communication device 10. Accordingly, the moving body 3 including the second optical communication device 20 can be reliably moved to the recommended relative position 41 of at least the second optical communication device 20 relative to the first optical communication device 10 in optical communication by the operation. Consequently, good optical communication can be reliably performed between the second optical communication device 20 and the first optical communication device 10. In addition, the moving body 3 including the second optical communication device 20 can be prevented from entering the unrecommended relative position 42 of the second optical communication device 20 relative to the first optical communication device 10 in optical communication by the operation. Consequently, it is possible to further prevent deterioration of communication quality and occurrence of interruption of communication between the second optical communication device 20 and the first optical communication device 10. Therefore, it is possible to further reduce influence of disturbance light such as sunlight during optical communication.

Also, in this embodiment, as described above, the underwater optical communication system further includes a first illuminance detector 15 and a second illuminance detector 24 being arranged to detect illuminances of the disturbance light in the plurality of different directions, and transmitting the detected illuminances of the disturbance light in the plurality of different directions to the control device 30 through the first optical communication device 10 and the second optical communication device 20. Accordingly, since the first illuminance detector 15 and the second illuminance detector 24 can detect intensities of illuminances (light intensities) of the disturbance light in a plurality of specified directions, it is possible to correctly acquire the disturbance-light-influence direction 43 of disturbance light, which can cause deterioration of communication quality and occurrence of interruption of communication. Consequently, it is possible to more accurately acquire the recommended relative position 41 and the unrecommended relative position 42 of the second optical communication device 20 relative to the first optical communication device 10.

Also, in this embodiment, as described above, the underwater optical communication system further includes a first turbidity detector 16 and a second turbidity detector 25 for detecting underwater turbidity and transmitting the detected underwater turbidity to the control device 30 through the first optical communication device 10 and the second optical communication device 20. Accordingly, it is possible to acquire an area where optical communication between the first optical communication device 10 and the second optical communication device 20 can be performed through communication light, and an area where optical communication is difficult in consideration of turbidity in the water detected by the first turbidity detector 16 and turbidity in the water detected by the second turbidity detector 25. Consequently, it is possible to more accurately acquire the recommended relative position 41 and the unrecommended relative position 42 of the second optical communication device 20 relative to the first optical communication device 10.

Also, in this embodiment, as described above, the underwater optical communication system further includes a first tidal current detector 17 and a second tidal current detector 26 for detecting underwater tidal currents and transmitting the detected underwater tidal currents to the control device 30 through the first optical communication device 10 and the second optical communication device 20. Accordingly, since the first tidal current detector 17 and the second tidal current detector 26 detect the underwater tidal currents, it is possible to acquire a direction and a position of the moving body 3 that are unlikely to be affected by the tidal current, and a direction and a position of the moving body 3 are likely to be affected by the tidal current. Consequently, if the moving body 3 enters a place where a tidal current is fast, optical communication between the first optical communication device 10 and the second optical communication device 20 can be stably and continuously performed by moving the moving body 3 to a position where the moving body is unlikely to be affected by the tidal current or by keeping an orientation of the moving body 3 by bringing the orientation of the moving body 3 into agreement with a direction of the tidal current.

Also, in this embodiment, as described above, the control device 30 controls, if the bidirectional optical communication between the first optical communication device 10 and the second optical communication device 20 is interrupted, movement of the second optical communication device 20 to a position that is set to allow the bidirectional optical communication between the first optical communication device 10 and the second optical communication device. Accordingly, since the second optical communication device 20 is automatically moved to a position that is set to allow the bidirectional optical communication between the first optical communication device 10 and the second optical communication device even if the bidirectional optical communication between the first optical communication device 10 and the second optical communication device 20 is interrupted, bidirectional optical communication between the first optical communication device 10 and the second optical communication device 20 can be resumed.

In this embodiment, as described above, the control device 30 includes the display 35; and the control device acquires positional information including absolute positional information on the first optical communication device 10 and relative positional information on the second optical communication device 20 relative to the first optical communication device 10, and indicates a position of the second optical communication device 20, the recommended relative position 41 and the unrecommended relative position 42 of the second optical communication device 20 relative to the first optical communication device 10 in the optical communication on the display 35. Accordingly, since the position of the second optical communication device 20 can be accurately acquired based on the absolute position information on the first optical communication device 10 and the relative position information on the second optical communication device 20 relative to the first optical communication device 10, and the position of the second optical communication device 20, and the recommended relative position 41 and the unrecommended relative position 42 of the second optical communication device 20 relative to the first optical communication device 10 in the optical communication are indicated on the display 35, the recommended relative position 41 and the unrecommended relative position 42 of the second optical communication device 20 relative to the first optical communication device 10 can be visually easily recognized.

Also, in this embodiment, as described above, the control device 30 constructs a virtual three-dimensional space 40 on which a real-time real environment is reflected, and presents the recommended relative position 41 and the unrecommended relative position 42 of the second optical communication device 20 relative to the first optical communication device 10 in the optical communication in the virtual three-dimensional space 40. Accordingly, since the recommended relative position 41 and the unrecommended relative position 42 of the second optical communication device 20 relative to the first optical communication device 10 in the optical communication is presented in the virtual three-dimensional space 40 on which a real-time real environment is reflected, the recommended relative position 41 and the unrecommended relative position 42 of the second optical communication device 20 relative to the first optical communication device 10 can be accurately presented closer to reality, and the recommended relative position 41 and the unrecommended relative position 42 of the second optical communication device 20 relative to the first optical communication device 10 can be more easily recognized.

Also, in this embodiment, as described above, the control device 30 is configured to move, if receiving an input of a position to which the second optical communication device 20 is to be moved in the virtual three-dimensional space 40 based on the presented at least one of the recommended relative position 41 and the unrecommended relative position 42 of the second optical communication device 20 relative to the first optical communication device 10, the second optical communication device 20 based on the input position. Also, in this embodiment, as described above, the underwater optical communication method further includes receiving an input for moving at least the second optical communication device 20 based on the presented the recommended relative position 41 and the unrecommended relative position 42 of the second optical communication device 20 relative to the first optical communication device 10; and moving at least the second optical communication device 20 based on the input for moving the second optical communication device 20. Accordingly, the moving body 3 including the second optical communication device 20 can be reliably moved to the recommended relative position 41 of at least the second optical communication device 20 relative to the first optical communication device 10 in optical communication by users. Consequently, good optical communication can be reliably performed between the second optical communication device 20 and the first optical communication device 10. In addition, the moving body 3 including the second optical communication device 20 can be prevented from entering the unrecommended relative position 42 of the second optical communication device 20 relative to the first optical communication device 10 in optical communication by users. Consequently, it is possible to further prevent deterioration of communication quality and occurrence of interruption of communication between the second optical communication device 20 and the first optical communication device 10. Therefore, it is possible to further reduce influence of disturbance light such as sunlight during optical communication.

### [Modified Embodiments]

Note that the embodiment disclosed this time must be considered as illustrative in all points and not restrictive. The scope of the present invention is not shown by the above description of the embodiments but by the scope of claims for patent, and all modifications (modified examples) within the meaning and scope equivalent to the scope of claims for patent are further included.

While the example in which the underwater optical communication system includes one first optical communication device and one second optical communication device has been shown in the aforementioned embodiment, the present invention is not limited to this. For example, the underwater optical communication system may include a plurality of first optical communication devices, or a plurality of second optical communication devices.

While the example in which the first optical communication device is provided to the fixed body has been shown in the aforementioned embodiment, the present invention is not limited to this. For example, the first optical communication device may be provided to a moving body moving in the water, or it may be suspended from and supported by a structure arranged on or above the water.

While the example in which the control device presents both the recommended relative position and the unrecommended relative position of the second optical communication device relative to the first optical communication device has been shown in the aforementioned embodiment, the present invention is not limited to this. For example, the control device may be configured to present at least one of the recommended relative position and the unrecommended relative position of the second optical communication device relative to the first optical communication device. Also, the control device may be configured to present at least one of the recommended relative position and the unrecommended relative position of the first optical communication device relative to the second optical communication device.

While the example in which the
recommended/unrecommended position acquirer acquires as the recommended relative position of the second optical communication device relative to the first optical communication device a position that satisfies conditions that an angle that is formed by the disturbance-light-influence direction and a direction of the second optical communication device relative to the first optical communication device falls within a set range, that the signal intensity of the communication light is not smaller than a set value, and that an angle that is formed by a direction of the tidal current and a moving direction of the moving body falls within a set range has been shown in the aforementioned embodiment, the present invention is not limited to this. For example, the
recommended/unrecommended position acquirer may be configured to acquire the recommended relative position of the second optical communication device relative to the first optical communication device by using a rule-based model based on the disturbance-light-influence direction in the virtual three-dimensional space, the signal intensity of the communication light at the coordinates of the virtual three-dimensional space, and a tidal current in the virtual three-dimensional space. Also, the recommended/unrecommended position acquirer may be configured to acquire the recommended relative position of the second optical communication device relative to the first optical communication device by using AI (artificial intelligence) based on the disturbance-light-influence direction in the virtual three-dimensional space, the signal intensity of the communication light at the coordinates of the virtual three-dimensional space, and a tidal current in the virtual three-dimensional space.

While the example in which the
recommended/unrecommended position acquirer acquires the recommended relative position of the second optical communication device relative to the first optical communication device based on the disturbance-light-influence direction in the virtual three-dimensional space, the signal intensity of the communication light at the coordinates of the virtual three-dimensional space, and a tidal current in the virtual three-dimensional space has been shown in the aforementioned embodiment, the present invention is not limited to this. For example, the recommended/unrecommended position acquirer may be configured to acquire the recommended relative position of the second optical communication device relative to the first optical communication device based only on the disturbance-light-influence direction in the virtual three-dimensional space, or the recommended/unrecommended position acquirer may be configured to acquire the recommended relative position of the second optical communication device relative to the first optical communication device based on the disturbance-light-influence direction in the virtual three-dimensional space, and at least one of the signal intensity of the communication light at the coordinates of the virtual three-dimensional space and a tidal current in the virtual three-dimensional space.

While the example in which the virtual three-dimensional space constructor acquires the disturbance-light-influence direction in the virtual three-dimensional space based on the intensities of illuminances (light intensities) of the disturbance light such as sunlight detected by the first and second illuminance detectors has been shown in the aforementioned embodiment, the present invention is not limited to this. For example, the virtual three-dimensional space constructor may be configured to acquire weather information from the outside sources, and to acquire the disturbance-light-influence direction of disturbance light such as sunlight from the weather information acquired. Also, the virtual three-dimensional space constructor may be configured to acquire time information from the outside sources, and to acquire the disturbance-light-influence direction of disturbance light such as sunlight based on positional information on the first optical communication device and the second optical communication device, and the time information acquired.

While the example in which the first optical communication device has the first to fourth areas divided, each of the first to fourth areas includes one first light emitter and one first light receiver, each of the first to fourth areas has four windows, and output ends of optical fibers extending from the first light emitter and input ends of optical fibers extending from the first light receiver are arranged in each of the four windows has been shown in the aforementioned embodiment, the present invention is not limited to this. The number of areas to be divided in the first optical communication device is not limited to a particular number but may be 8 or 16. Also, the number of windows included in each area is not limited to a particular number. Also, the second optical communication device may have first to fourth areas divided; each of the first to fourth areas may include one second light emitter and one second light receiver; each of the first to fourth areas may have four windows; and output ends of optical fibers extending from the second light emitter and input ends of optical fibers extending from the second light receiver may be arranged in each of the four windows.

While the example in which the first illuminance detector, the first turbidity detector, and the first tidal current detector are included in the moving body has been shown in the aforementioned embodiment, the present invention is not limited to this. For example, the first illuminance detector, the first turbidity detector, and the first tidal current detector may be provided to a structure other than the moving body in the water. Also, one of the first turbidity detector and the first tidal current detector may be omitted.

While the example in which the second illuminance detector, the second turbidity detector, and the second current detector are included in the moving body has been shown in the aforementioned embodiment, the present invention is not limited to this. For example, the second illuminance detector, the second turbidity detector, and the second tidal current detector may be provided to a structure other than the moving body in the water. Also, one of the second turbidity detector and the second tidal current detector may be omitted.

While the example in which the control device is configured to move the second optical communication device to a position that is operated based on the presented at least one of the recommended relative position and the unrecommended relative position of at least the second optical communication device relative to the first optical communication device has been shown in the aforementioned embodiment, the present invention is not limited to this. For example, the control device may be configured to determine a position of the moving body based on the presented at least one of the recommended relative position and the unrecommended relative position of at least the second optical communication device relative to the first optical communication device, and to move the second optical communication device to the position determined.

### [Modes]

The aforementioned exemplary embodiment will be understood as concrete examples of the following modes by those skilled in the art.

### (Mode Item 1)

An underwater optical communication system according to mode item 1 includes a first optical communication device being arranged underwater, and including a first light emitter and a first light receiver; a second optical communication device being provided to a moving body for moving underwater, including a second light emitter and a second light receiver and performing bidirectional optical communication between the second optical communication device and the first optical communication device; and a control device for presenting at least one of a recommended relative position and an unrecommended relative position of at least the second optical communication device relative to the first optical communication device in the optical communication based on disturbance light.

### (Mode Item 2)

In the underwater optical communication system according to mode item 1, the control device presents the at least one of the recommended relative position and the unrecommended relative position of at least the second optical communication device relative to the first optical communication device in the optical communication based on light intensities of the disturbance light in a plurality of directions different from each other.

### (Mode Item 3)

In the underwater optical communication system according to mode item 1 or 2, the control device is configured to move the second optical communication device to a position that is operated or determined based on the presented at least one of the recommended relative position and the unrecommended relative position of at least the second optical communication device relative to the first optical communication device.

### (Mode Item 4)

In the underwater optical communication system according to any of mode items 1 to 3, an illuminance detector is further provided being arranged to detect illuminances of the disturbance light in the plurality of different directions, and transmitting the detected illuminances of the disturbance light in the plurality of different directions to the control device through at least one of the first optical communication device and the second optical communication device.

### (Mode Item 5)

In the underwater optical communication system according to any of mode items 1 to 4, a turbidity detector is further provided for detecting underwater turbidity and transmitting the detected underwater turbidity to the control device through at least one of the first optical communication device and the second optical communication device.

### (Mode Item 6)

In the underwater optical communication system according to any of mode items 1 to 5, a tidal current detector is further provided for detecting an underwater tidal current and transmitting the detected tidal current to the control device through at least one of the first optical communication device and the second optical communication device.

### (Mode Item 7)

In the underwater optical communication system according to any of mode items 1 to 6, the control device controls, if the bidirectional optical communication between the first optical communication device and the second optical communication device is interrupted, movement of the second optical communication device to a position that is set to allow the bidirectional optical communication between the first optical communication device and the second optical communication device.

### (Mode Item 8)

In the underwater optical communication system according to any of mode items 1 to 7, the control device includes a display; and the control device acquires positional information including absolute positional information on the first optical communication device and relative positional information on the second optical communication device relative to the first optical communication device, and indicates a position of the second optical communication device, and the at least one of the recommended relative position and the unrecommended relative position of at least the second optical communication device relative to the first optical communication device in the optical communication on the display.

### (Mode Item 9)

In the underwater optical communication system according to any of mode items 1 to 8, the control device constructs a virtual three-dimensional space on which a real-time real environment is reflected, and presents the at least one of the recommended relative position and the unrecommended relative position of at least the second optical communication device relative to the first optical communication device in the optical communication in the virtual three-dimensional space.

### (Mode Item 10)

In the underwater optical communication system according to mode item 9, the control device is configured to move, if receiving an input of a position to which the second optical communication device is to be moved in the virtual three-dimensional space based on the presented at least one of the recommended relative position and the unrecommended relative position of the second optical communication device relative to the first optical communication device, the second optical communication device based on the input position.

### (Mode Item 11)

An underwater optical communication method for performing optical communication between a first optical communication device and a second optical communication device underwater according to mode item 11, the method including acquiring information on disturbance light; and presenting at least one of a recommended relative position and an unrecommended relative position of at least the second optical communication device relative to the first optical communication device based on the acquired information on the disturbance light.

### (Mode Item 12)

In the underwater optical communication method according to mode item 11, receiving an input for moving at least the second optical communication device based on the presented at least one of the recommended relative position and the unrecommended relative position of the second optical communication device relative to the first optical communication device; and moving at least the second optical communication device based on the input for moving at least the second optical communication device are further provided.

## Claims

1. An underwater optical communication system (1) comprising:
a first optical communication device (10) being arranged underwater, and including a first light emitter (11) and a first light receiver (12);
a second optical communication device (20) being provided to a moving body (3) for moving underwater, including a second light emitter (21) and a second light receiver (22) and performing bidirectional optical communication between the second optical communication device and the first optical communication device (10); and
a control device (30) for presenting at least one of a recommended relative position (41) and an unrecommended relative position (42) of at least the second optical communication device (20) relative to the first optical communication device (10) in the optical communication based on disturbance light.

2. The underwater optical communication system (1) according to claim 1, wherein the control device (30) presents the at least one of the recommended relative position (41) and the unrecommended relative position (42) of at least the second optical communication device (20) relative to the first optical communication device (10) in the optical communication based on light intensities of the disturbance light in a plurality of directions different from each other.

3. The underwater optical communication system (1) according to claim 1, wherein the control device is configured to move the second optical communication device (20) to a position that is operated or determined based on the presented at least one of the recommended relative position (41) and the unrecommended relative position (42) of at least the second optical communication device (20) relative to the first optical communication device (10).

4. The underwater optical communication system (1) according to claim 1 further comprising an illuminance detector (15, 24) being arranged to detect illuminances of the disturbance light in a plurality of different directions, and transmitting the detected illuminances of the disturbance light in the plurality of different directions to the control device (30) through at least one of the first optical communication device (10) and the second optical communication device (20).

5. The underwater optical communication system (1) according to claim 1 further comprising a turbidity detector (16, 25) for detecting underwater turbidity and transmitting the detected underwater turbidity to the control device (30) through at least one of the first optical communication device (10) and the second optical communication device (20).

6. The underwater optical communication system (1) according to claim 1 further comprising a tidal current detector (17, 26) for detecting an underwater tidal current and transmitting the detected tidal current to the control device (30) through at least one of the first optical communication device (10) and the second optical communication device (20).

7. The underwater optical communication system (1) according to claim 1, wherein the control device (30) controls, if the bidirectional optical communication between the first optical communication device (10) and the second optical communication device (20) is interrupted, movement of the second optical communication device (20) to a position that is set to allow the bidirectional optical communication between the first optical communication device (10) and the second optical communication device.

8. The underwater optical communication system (1) according to claim 1, wherein
the control device (30) includes a display (35); and
the control device acquires positional information including absolute positional information on the first optical communication device (10) and relative positional information on the second optical communication device (20) relative to the first optical communication device (10), and indicates a position of the second optical communication device (20), and the at least one of the recommended relative position (41) and the unrecommended relative position (42) of at least the second optical communication device (20) relative to the first optical communication device (10) in the optical communication on the display (35).

9. The underwater optical communication system (1) according to any of claims 1 to 8, wherein the control device (30) constructs a virtual three-dimensional space (40) on which a real-time real environment is reflected, and presents the at least one of the recommended relative position (41) and the unrecommended relative position (42) of at least the second optical communication device (20) relative to the first optical communication device (10) in the optical communication in the virtual three-dimensional space (40).

10. The underwater optical communication system (1) according to claim 9, wherein the control device (30) is configured to move, if receiving an input of a position to which the second optical communication device (20) is to be moved in the virtual three-dimensional space (40) based on the presented at least one of the recommended relative position (41) and the unrecommended relative position (42) of the second optical communication device (20) relative to the first optical communication device (10), the second optical communication device (20) based on the input position.

11. An underwater optical communication method for performing optical communication between a first optical communication device (10) and a second optical communication device (20) underwater, the method comprising
acquiring information on disturbance light; and
presenting at least one of a recommended relative position (41) and an unrecommended relative position (42) of at least the second optical communication device (20) relative to the first optical communication device (10) based on the acquired information on the disturbance light.

12. The underwater optical communication method according to claim 11 further comprising
receiving an input for moving at least the second optical communication device (20) based on the presented at least one of the recommended relative position (41) and the unrecommended relative position (42) of the second optical communication device (20) relative to the first optical communication device (10); and
moving at least the second optical communication device (20) based on the input for moving at least the second optical communication device (20).
